# EUROPÄISCHE PATENTANMELDUNG

(11) **EP 2 168 490 A1**
(43) Veröffentlichungstag der Anmeldung: **31.03.2010**
(21) Anmeldenummer: 09154848.7
(22) Anmeldetag: 11.03.2009
(51) Int. Cl.: A61B 6/03, A61B 6/00

(54) **Röntgengerät zur Brustuntersuchung mit einer Detektor-Röhren Anordnung für hochauflösende Aufnahmen**

(30) Priorität: 29.09.2008 DE 102008042430
(71) Anmelder: MIR Medical Imaging Research Holding GmbH, 91096 Möhrendorf (DE)
(72) Erfinder: Kalender, Willi, 91096, Möhrendorf (DE); Schilling, Harry, 85072, Eichstätt (DE)
(74) Vertreter: Lohr, Georg

(57) **Zusammenfassung**

Ein Röntgengerät zur Abbildung einer weiblichen Brust hat eine Patientenliege (20) zur Aufnahme einer Patientin. Eine Spiral-CT Gantry (10) umfasst eine Röntgenröhre (15) und einen Detektor (50,60). Der Detektor ist so gegenüber der Röntgenröhre geneigt, dass die Mitte des Strahlenbündels aus der Röntgenröhre senkrecht auf den Detektor auftrifft. Weiterhin weist der Detektor eine Krümmung um die Rotationsachse der Gantry auf. Mit dieser Konfiguration lassen sich besonders Platz sparende und gleichzeitig besonders hoch auflösende Röntgengeräte bauen.

## Beschreibung

### Technisches Gebiet

Die Erfindung betrifft ein Röntgengerät zur Abbildung der weiblichen Brust (Mammografie). Insbesondere betrifft die Erfindung ein Gerät, mit dem hochauflösende Röntgenaufnahmen hergestellt werden können.

### Stand der Technik

Aus dem Stand der Technik sind Röntgengeräte mit der Röntgenröhre und einem großflächigen Detektor bekannt. In der US 6,480,565 B1 ist ein Röntgengerät offenbart, bei dem der Detektor in seiner Größe derart bemessen ist, dass die ganze Brust abgebildet werden kann. Um dreidimensionale Abbildungen zu erzeugen, befinden sich die Röntgenröhre und der Detektor auf einer drehbaren Gantry, welche Aufnahmen aus unterschiedlichen Winkelpositionen erlaubt. In der US 2007/0064867 ist ein Röntgengerät basierend auf einem Spiral-CT Scanner offenbart. Es werden hier kontinuierlich während der Drehung der Gantry mit Röntgenröhre und Detektor Aufnahmen gemacht. Allerdings ergibt sich durch die Anordnung von der Brustwand aus betrachtet ein relativ großer Bereich, der nicht abgebildet werden kann.

### Darstellung der Erfindung

Der Erfindung liegt die Aufgabe zugrunde, ein Röntgengerät zu gestalten, welches die weibliche Brust diagnostisch exakt, schnell und kostengünstig und mit hoher Auflösung abbildet. Ein weiterer Aspekt der Erfindung ist die Ausgestaltung eines Röntgendetektors für ein solches Röntgengerät.

Diese Aufgabe wird durch ein Röntgengerät nach Anspruch 1 oder 2 beziehungsweise durch einen Röntgendetektor nach Anspruch 8 gelöst. Vorteilhafte Ausgestaltungen der Erfindung sind in den Unteransprüchen angegeben.

Ein erfindungsgemäßes Röntgengerät zur Abbildung einer weiblichen Brust 31 umfasst eine Röntgenröhre 15 und einem Röntgendetektor 50, 60. Von der Röntgenröhre 15 wird ein Strahlenbündel ausgehend von einer Anode 51 durch die Brust 31 auf den Röntgendetektor 50, 60 emittiert. Dieses Strahlenbündel wird idealerweise so dimensioniert, dass es gerade die gesamte aktive Fläche des Röntgendetektors 50, 60 abdeckt. Nachfolgend wird nur noch auf ein solches Strahlenbündel Bezug genommen. Sollte das Strahlenbündel größer sein, so ist für die Erfindung nur der Anteil des Strahlenbündels relevant, der die aktive Detektorfläche abdeckt. Um Aufnahmen bis möglichst nahe an die Brustwand machen zu können, wird das Strahlenbündel möglichst nahe parallel zur Brustwand geführt. Da die Brustwand auf einer Auflagefläche, wie beispielsweise einer Patientenliege aufliegt, liegt diese näherungsweise parallel zur Auflagefläche 20. Entsprechend wird das Strahlenbündel vorzugsweise parallel zur Auflagefläche 20 geführt und/oder geometrisch durch eine Fläche parallel zu dieser Auflagefläche 20 begrenzt.

Für den Fall eines Röntgengerätes, bei dem die Röntgenröhre sowie der Röntgendetektor während der Aufnahme in festen Positionen stehen, weist das von der Röntgenröhre 15 emittierte Strahlenbündel 16 einen Winkel 2α (54) auf. Um nun Artefakte (Kegelstrahlartefakte) durch unterschiedliche Weglängen der Strahlung von der Röntgenröhre zum Detektor zu vermeiden, wird der Röntgendetektor um den Winkel α, also um den halben Winkel des Strahlenbündels, gegen die senkrechte auf eine Auflagefläche 20 für die Patientin, welche näherungsweise parallel zu Brustwand liegt, geneigt.

Für den Fall eines Röntgengerätes, bei eine die Röntgenröhre sowie der Röntgendetektor auf einer Gantry 10 angeordnet sind, welche um eine Rotationsachse 12 rotiert, ist der Röntgendetektor um den Winkel α gegen die Rotationsachse 12 der Gantry 10 geneigt angeordnet. Hier ist der Winkel α der halbe Winkel des von der Röntgenröhre 15 emittierten Strahlenbündels 16. Somit schneidet sich eine gedankliche Verlängerung der Fläche des Röntgendetektors 50, 60 mit der Rotationsachse 12 unterhalb der Auflagefläche mit dem Winkel α. In beiden Ausführungsformen des erfindungsgemäßen Röntgengerätes ergibt sich eine Anordnung, bei der der Zentralstrahl 52 senkrecht auf die Mitte des Röntgendetektors 50, 60 trifft. Mit dieser Anordnung sind die Weglängendifferenzen zwischen dem Zentralstrahl und den Strahlen am Rand des Detektors geringer als beim Stand der Technik.

Entsprechend einer weiteren Ausgestaltung der Erfindung sind die Röntgenröhre 15 und der Röntgendetektor 50, 60 auf einer drehbaren Gantry 10 angeordnet, welche in eine kontinuierliche Rotationsbewegung versetzt werden kann. Weiterhin kann diese mit der Drehung synchronisiert, parallel zur Rotationsachse linear verschoben werden. Diese Gantry entspricht der Gantry eines Spiral-Computertomographen.

In einer weiteren Ausführungsformen der Erfindung wird entsprechend das Strahlenbündel vorzugsweise parallel zur Auflagefläche 20 geführt und/oder geometrisch durch eine Fläche parallel zu dieser Auflagefläche 20 begrenzt.

In einer weiteren Ausgestaltung der Erfindung ist der Detektor gewölbt ausgeführt, so dass der Zentralstrahl 52 der Strahlenbündels und auch weitere seitlich oberhalb diesen verlaufenden Strahlen senkrecht auf die Fläche des Detektors auftreffen.

Ein weiterer Aspekt Erfindung betrifft einen Röntgendetektor 60, welcher ein gekachelter Runddetektor ist. Er umfasst einen gewölbten Detektorträger 78. Auf diesen Detektorträger 78 sind mittels Detektorhaltern 70 Detektorkacheln 61 befestigt. Bevorzugt überlappen sich diese Detektorkacheln geringfügig, um eine lückenlose Abbildung zu erreichen. Besonders günstig ist es, wenn sich einzelne Detektorkacheln dachziegelartig überlappen. Der Detektorträger 78 bevorzugt ist in seiner Wölbung derart angepasst, dass die darauf befestigten Detektorkacheln eine Wölbung mit einem Radius entsprechend dem Abstand des Detektors zur Röntgenröhre 15 haben. Ein solcher erfindungsgemäßer Röntgendetektor kann auch in einem erfindungsgemäßen Röntgengerät eingesetzt werden.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt die Gantry eines erfindungsgemäßen Röntgengerätes mit einem Flachbilddetektor.
Figur 2 zeigt ein Röntgengerät nach dem Stand der Technik.
Figur 3 zeigt die Winkelverhältnisse eines erfindungsgemäßen Röntgengerätes.
Figur 4 zeigt ein Röntgengerät mit einem Gewölbten Detektor.
Figur 5 zeigt ein erfindungsgemäßes Röntgengerät ausgeführt als Spiral-CT Scanner.
Figur 6 zeigt eine Draufsicht auf einen gekachelten Rund-Detektor.
Figur 7 zeigt einen Detektorhalter im Schnitt.
Figur 8 zeigt eine Draufsicht auf Detektorkacheln.
Figur 9 zeigt die Ansicht des gekachelten Runddetektors aus der Perspektive der Röntgenröhre.
Figur 10 zeigt die Ansicht des gekachelten Runddetektors mit mehreren Detektorreihen.
Figur 11 zeigt einen einstückigen Detektorträger.

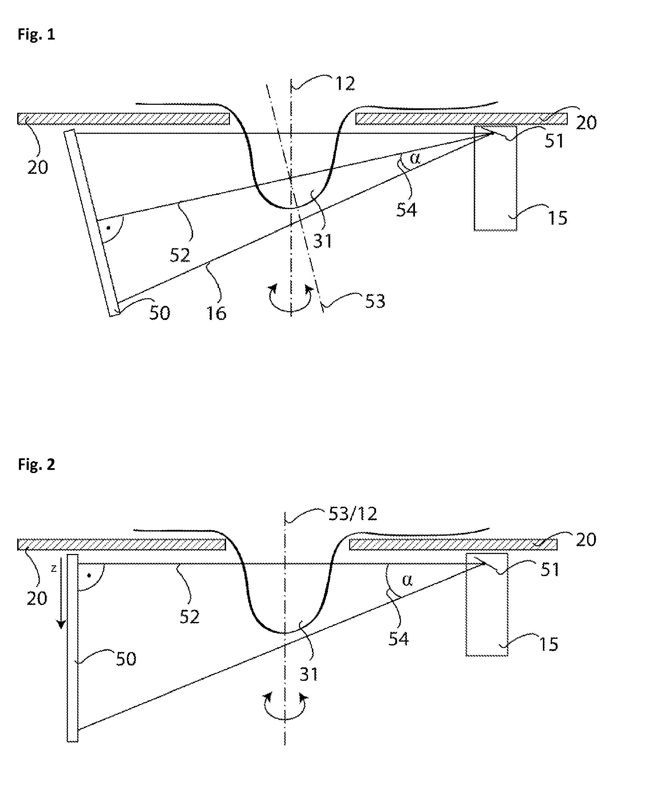

Figur 1 zeigt ein erfindungsgemäßes Röntgengerät mit einem Flachbilddetektor 50. Die zu untersuchende Brust 31 einer Patientin hängt durch eine Öffnung in einer Auflagefläche 20, welche hier als Patientenliege ausgeführt ist. Selbstverständlich kann diese Anordnung auch um beliebige Winkel gedreht werden, so dass die Patientenliege 20 auch schräg oder senkrecht steht und nur noch als eine Auflagefläche ausgeführt ist, durch welche die Brust hindurch gesteckt ist. Die Brustwand der Patienten soll möglichst nahe an der Patientenliege anliegen, so dass die Brust möglichst vollständig abgebildet werden kann. Zur Abbildung ist eine um die Rotationsachse 12 rotierende Gantry 10 mit einer Röntgenröhre 15 und einem Flachbilddetektor 50 vorgesehen. Die Röntgenröhre 15 erzeugt ein Strahlenbündel mittels der Anode 51, welches die Brust 31 durchdringt und von dem Flachbilddetektor 50 aufgenommen wird. Idealerweise ist das Strahlenbündel auf die aktive Fläche des Detektors begrenzt. So soll es keinesfalls die Patientenliege 20 durchdringen. Das halbe Strahlenbündel hat einen Winkel α (54). Das ganze Strahlenbündel hat einen Winkel 2α. Der Flachbilddetektor 50 ist gegenüber der Rotationsachse 12 um einen Winkel α in Richtung zur Röntgenröhre verkippt. Daraus resultiert, dass der Zentralstrahl 52 in der Mitte des Strahlenbündels senkrecht auf die Oberfläche des Flachbilddetektors 50 in der Mitte des Flachbilddetektors 50 auftrifft. Somit liegt die Zentralachse 53 des Strahlenbündels, die als Senkrechte auf den Zentralstrahl definiert ist, ebenfalls unter einem Winkel α zur Rotationsachse 12. Selbstverständlich handelt es sich bei dem Detektor um ein flächiges Gebilde, welches noch eine Ausdehnung senkrecht zur Zeichenebene hat. Der Begriff der Mitte des Flachbilddetektors 50 bezieht sich hier auf die Mitte seiner Dimension parallel zur Zeichenebene. Durch diese Ausgestaltung sind die Weglängenunterschiede zwischen dem Zentralstrahl und den äußeren Strahlen am Rand des Flachbilddetektors 50 geringer als beim Stand der Technik.

Figur 2 zeigt ein Röntgengerät nach dem Stand der Technik. Die Anordnung entspricht weitgehend derjenigen aus Figur 1. Allerdings ist hier der Flachbilddetektor 50 in Richtung der Z-Achse parallel zur Rotationsachse 12 beziehungsweise senkrecht zur Patientenliege 20 angeordnet. Durch die unterschiedlichen Weglängen von kürzestem Strahl, in diesem Falle dem Zentralstrahl 52, welcher den Flachbilddetektor 50 an seinem oberen Ende senkrecht trifft, bis hin zum längsten Strahl an der Unterseite des Detektors 50 ergeben sich deutliche Artefakte. Allerdings ist die Bildauswertung hier besonders einfach, da die Zentralachse 53 parallel zur Rotationsachse 12 verläuft.

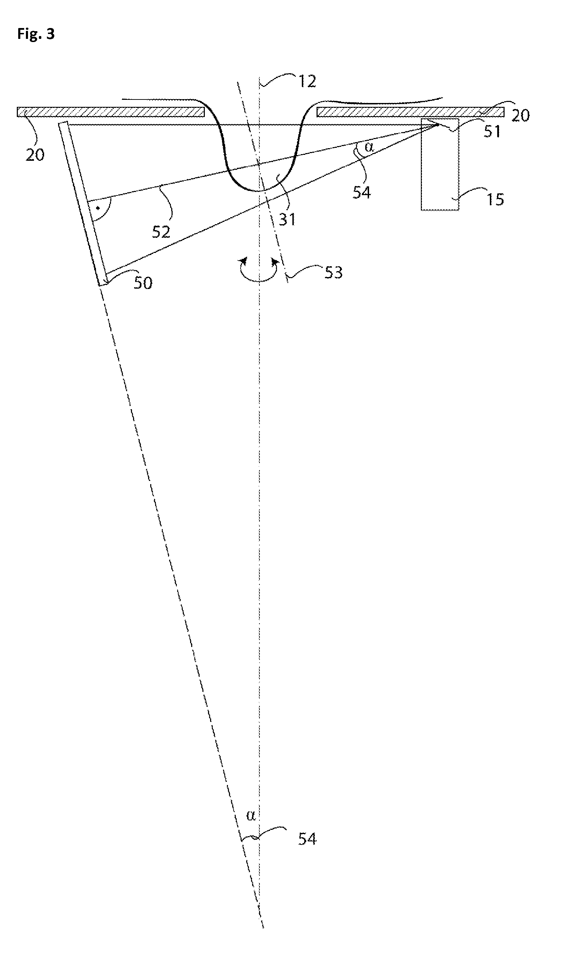

Figur 3 zeigt die Winkelverhältnisse des erfindungsgemäßen Röntgengerätes. Verlängert man gedanklich die Fläche des Flachbilddetektors 50, so schneidet sich diese Verlängerungslinie unter einem Winkel α (54) mit der Rotationsachse 12.

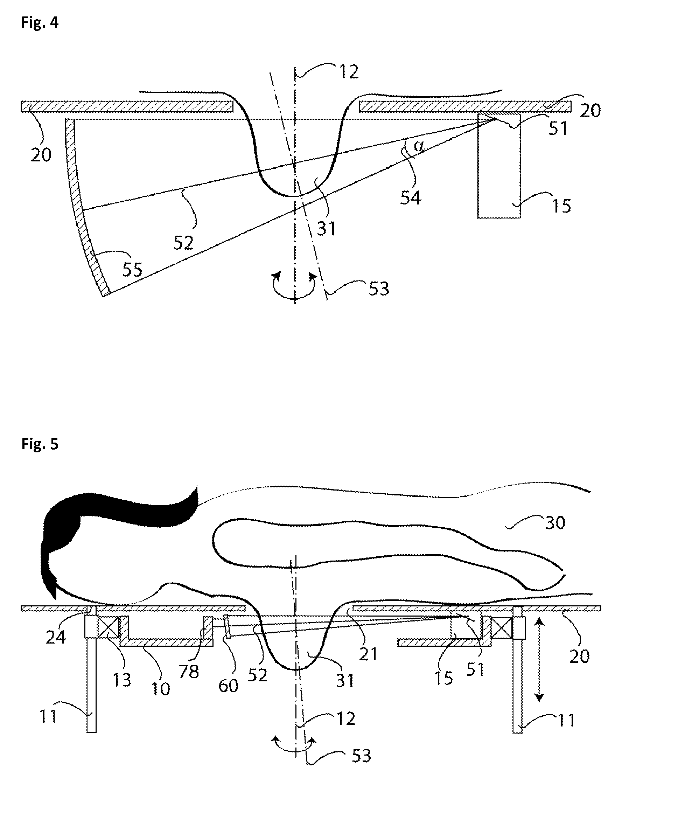

Figur 4 zeigt ein Röntgengerät mit einem gewölbten Detektor. Durch die Wölbung ist der Detektor noch besser angepasst. So tritt nicht nur der Zentralstrahl 52, sondern auch alle anderen Strahlen unter einem senkrechten Winkel auf den Detektor auf. Damit lassen sich die Artefakte weiter minimieren.

Figur 5 zeigt ein erfindungsgemäßes Röntgengerät ausgeführt als Spiral-CT Scanner. Eine Gantry 10 nimmt hier eine Röntgenröhre 15 sowie einen gekachelten Rund-Detektor 60 auf einem Detektorträger 78 auf. Die Gantry 10 kann zur Abbildung der Brust 31 in eine kontinuierliche Rotationsbewegung versetzt werden. Weiterhin kann diese mit der Drehung synchronisiert, senkrecht zur Rotationsachse linear verschoben werden. Dadurch, dass die Brust nun in einzelnen Spiralen abgetastet wird, kann der Detektor 60 wesentlich kompakter ausgeführt sein. Somit kann der Detektor 60 auch wesentlich näher an die Patientenliege 20 herangeführt werden. Damit kann ein größerer Bereich der Brust 31 nahe der Brustwand abgebildet werden.

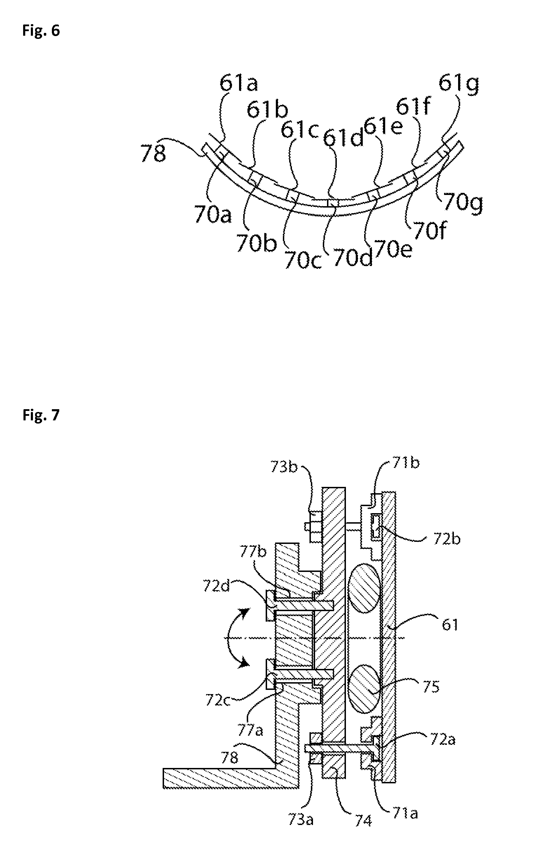

Figur 6 zeigt eine Draufsicht auf einen gekachelten Rund-Detektor 60. Dieser Detektor hat einen Detektorträger 78, auf dem eine Vielzahl von ebenen Detektorkacheln 61a - 61g angebracht sind. Diese Detektorkacheln umfassen jeweils wenigstens einen hochauflösenden Halbleiter-Röntgendetektor. Der Detektorträger 78 gibt die gewölbte Grundform des Detektors vor. Vorzugsweise überlappen sich die Detektorkacheln geringfügig, so dass die Anschlussbereiche am Rand einer Kachel unter der benachbarten Kachel liegen. Damit ist eine lückenlose Abbildung möglich. Die einzelnen Detektorkacheln 61a - 61g sind mit Detektorhaltern 70a - 70g an dem Detektorträger 78 befestigt. Die Detektorhalter sind vorzugsweise justierbar ausgeführt. Durch diese Detektorhalter kann eine einfache Justage der Anordnung erfolgen.

Figur 7 zeigt einen Detektorhalter 70 im Schnitt. Der Detektorhalter verbindet eine Detektorkachel 61 mit dem Detektorträger 78. hierzu ist eine Spanplatte 74 mittels der Schrauben 72 an dem Detektorträger 78 befestigt. Diese Spannplatte lässt sich aufgrund der Langlöcher 77 geringfügig gegenüber dem Detektorträger drehen. Zwischen der Detektorkachel und der Spannplatte ist ein Gummiring 75, vorzugsweise ein O-Ring eingesetzt. Gegen den elastischen Gummiring wird die Detektorkachel 61 mittels weiterer Schrauben, welche durch ein Halteprofil 71 an der Detektorkachel befestigt sind und den Muttern 73 verspannt. Es werden hier vorzugsweise drei Schrauben eingesetzt, so dass die Detektorkachel 61 in alle Richtungen ausgerichtet werden kann. Nach der Montage kann der Detektorhalter beispielsweise durch Klebstoff fixiert werden. Besonders bevorzugt ist es, wenn hierbei ein Zweikomponentenkleber in den Detektorhalter eingebracht wird.

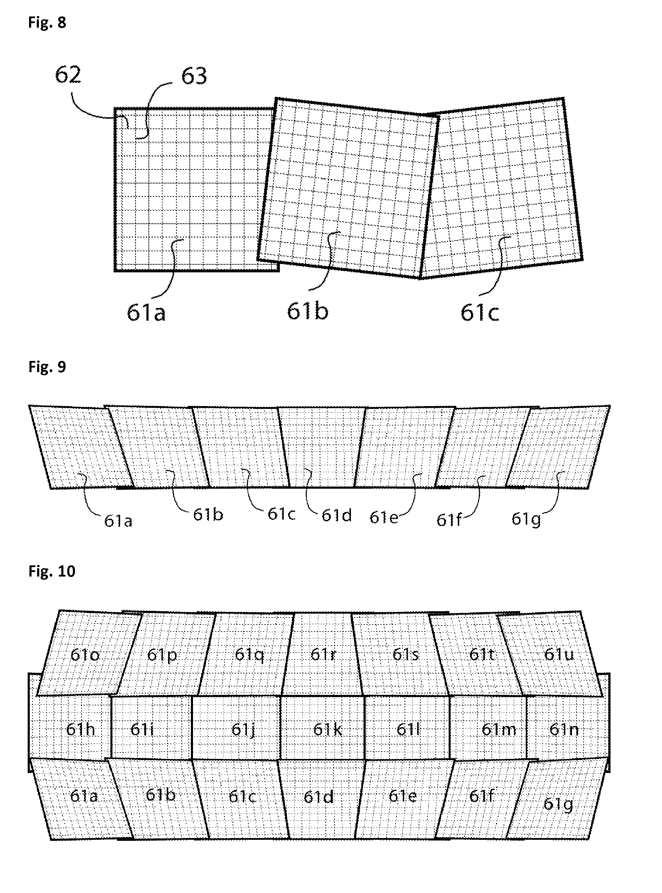

Figur 8 zeigt eine Draufsicht auf Detektorkacheln 61 des gekachelten Runddetektors 60. Diese Kacheln weisen jeweils in dieser Darstellung horizontal verlaufende Detektorzeilen 62 und vertikal verlaufende Detektorspalten 63 aus einzelnen Detektorelementen auf. Die Größe eines solchen Detektorelements liegt typischerweise in einem Bereich von 10 Mikrometer bis 500 Mikrometer, bevorzugt bei 100 Mikrometer. Da die einzelnen Detektorkacheln Fertigungstoleranzen haben, müssen diese individuell ausgerichtet beziehungsweise positioniert werden. Zudem müssen sich die Detektorkacheln geringfügig überlappen, da am Rande der einzelnen Detektorkacheln Anschlusselemente (hier nicht dargestellt) vorhanden sind.

Figur 9 zeigt die Ansicht des gekachelten Runddetektors 60 aus der Perspektive der Röntgenröhre 15. Hierin sind die schräg gestellten Detektorkacheln 61a - 61g in der perspektivischen Darstellung gut zu erkennen. Weiterhin ist die dachziegelartige Überlappung erkennbar.

Figur 10 zeigt die Ansicht eines weiteren gekachelten Runddetektors 60 aus der Perspektive der Röntgenröhre 15. Hierin sind drei übereinander angeordnete Reihen aus Detektorkacheln vorgesehen. Die obere Reihe mit dem Detektorkacheln 61o - 61u ist gegenüber der mittleren Reihe mit dem Detektorkacheln 61h - 61n verkippt. Gleiches gilt für die untere Reihe mit dem Detektorkacheln 61a - 61g. Durch die verkippte obere und untere Reihe ergibt sich eine Annäherung an eine gewölbte Bauform

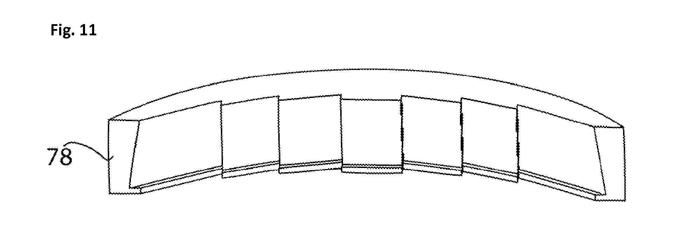

Figur 11 zeigt einen Detektorträger, welcher beispielsweise aus Aluminium hergestellt ist. Dieser Träger weist Aufnahmeflächen zur Befestigung der einzelnen Detektorkacheln auf.

### Bezugszeichenliste

- 10: Gantry
- 11: Gantryhubantrieb
- 12: Rotationsachse
- 15: Röntgenröhre
- 16: Strahlenbündel
- 20: Auflagefläche
- 22: Patientenliegenhubantrieb
- 30: Patientin
- 31: Brust
- 50: Flachbilddetektor
- 51: Anode
- 52: Zentralstrahl
- 53: Zentralachse
- 54: Winkel des halben Strahlenbündels
- 55: Gewölbter Detektor
- 60: Gekachelter Rund-Detektor
- 61: Detektorkacheln
- 62: Detektorzeile
- 63: Detektorspalte
- 70: Detektorhalter
- 71: Halteprofil
- 72: Schraube
- 73: Mutter
- 74: Spannplatte
- 75: Gummiring
- 77: Langloch
- 78: Detektorträger

## Patentansprüche

1. Röntgengerät zur Abbildung einer weiblichen Brust (31) einer Patientin (30), umfassend eine Auflagefläche (20) für die Patientin, sowie eine um eine Rotationsachse (12) rotierende Gantry (10), umfassend
- eine Röntgenröhre (15) zur Emission eines Strahlenbündels (16), ausgehend von einer Anode (51), welches einen Öffnungswinkel 2α (54) aufweist, und
- einen Röntgendetektor (50, 60) zur Detektion der von der Röntgenröhre (15) emittierten Strahlung,
wobei die Brust (31) zwischen der Röntgenröhre (15) und dem Röntgendetektor (50, 60) angeordnet ist,
**dadurch gekennzeichnet, dass**
der Röntgendetektor (50, 60) um einen Winkel α (54) gegen die Rotationsachse (12) geneigt ist, so dass der Zentralstrahl (52) des Strahlenbündels (16) unter einem rechten Winkel auf die Mitte des Röntgendetektors (50, 60) trifft.

2. Röntgengerät zur Abbildung einer weiblichen Brust (31) einer Patientin (30), umfassend eine Auflagefläche (20) für die Patientin, umfassend
- eine Röntgenröhre (15) zur Emission eines Strahlenbündels (16), ausgehend von einer Anode (51), welches einen Öffnungswinkel 2α (54) aufweist, und
- einen Röntgendetektor (50, 60) zur Detektion der von der Röntgenröhre (15) emittierten Strahlung,
wobei die Brust (31) zwischen der Röntgenröhre (15) und dem Röntgendetektor (50, 60) angeordnet ist,
**dadurch gekennzeichnet, dass**
der Röntgendetektor (50, 60) um einen Winkel α (54) gegen die senkrechte zur Auflagefläche (20) geneigt ist, so dass der Zentralstrahl (52) des Strahlenbündels (16) unter einem rechten Winkel auf die Mitte des Röntgendetektors (50, 60) trifft.

3. Röntgengerät nach Anspruch 1,
**dadurch gekennzeichnet, dass**
die Gantry (10) zur Abbildung der Brust (31) in eine kontinuierliche Rotationsbewegung versetzt werden kann, und mit der Drehung synchronisiert, senkrecht zur Rotationsachse linear verschoben werden kann.

4. Röntgengerät nach Anspruch 1 oder 2,
**dadurch gekennzeichnet, dass**
Strahlenbündel durch eine Fläche parallel zur Auflagefläche (20) begrenzt ist.

5. Röntgengerät nach Anspruch 1 oder 2,
**dadurch gekennzeichnet, dass**
der Röntgendetektor gewölbt ist.

6. Röntgengerät nach Anspruch 1 oder 2,
**dadurch gekennzeichnet, dass**
der Röntgendetektor (60) ein gekachelter Runddetektor ist, welcher einen gewölbten Detektorträger (78) umfasst, auf dem mittels Detektorhaltern (70) Detektorkacheln (61) befestigt sind.

7. Röntgengerät nach Anspruch 6,
**dadurch gekennzeichnet, dass**
die Detektorkacheln (61) sich teilweise überlappen.

8. Röntgendetektor (60) für ein Röntgengerät zur Abbildung einer weiblichen Brust (31) einer Patientin (30),
**dadurch gekennzeichnet, dass**
der Röntgendetektor (60) ein gekachelter Runddetektor ist, welcher einen gewölbten Detektorträger (78) umfasst, auf dem mittels Detektorhaltern (70) Detektorkacheln (61) befestigt sind.

9. Röntgendetektor nach Anspruch 8,
**dadurch gekennzeichnet, dass**
die Detektorkacheln (61) sich teilweise überlappen.
